# EUROPÄISCHE PATENTSCHRIFT

(11) **EP 3 943 071 B1**
(45) Veröffentlichungstag und Bekanntmachung des Hinweises auf die Patenterteilung: **18.06.2025**
(21) Anmeldenummer: 21000088.1
(22) Anmeldetag: 25.03.2021
(51) Int. Cl.: A61K 9/14, A61K 47/10, A61K 47/34, A61K 47/40, A61K 47/44, A61P 9/00, A61P 29/00

(54) **ZUSAMMENSETZUNG, ENTHALTEND NATÜRLICHE LIPOPHILE VERBINDUNGEN, VERWENDUNG DER ZUSAMMENSETZUNG UND VERFAHREN ZUR HERSTELLUNG DER ZUSAMMENSETZUNG**
COMPOSITION COMPRISING NATURAL LIPOPHILIC COMPOUNDS, USE OF THE COMPOSITION AND METHOD FOR PREPARING THE COMPOSITION
COMPOSITION CONTENANT DES COMPOSÉS LIPOPHILES NATURELS, UTILISATION DE LA COMPOSITION ET PROCÉDÉ DE PRÉPARATION DE LA COMPOSITION

(30) Priorität: 31.03.2020 DE 102020002031
(43) Veröffentlichungstag der Anmeldung: 26.01.2022
(73) Patentinhaber: Khinikadze, Zurab Durmischchanowitch, 143442 Angelovo (RU); Kasch, Tengis, 10713 Berlin (DE); Hartmann, Bert Uwe, 12527 Berlin (DE)
(72) Erfinder: Khinikadze, Zurab Durmischchanowitch, 143442 Angelovo (RU); Kasch, Tengis, 10713 Berlin (DE); Hartmann, Bert Uwe, 12527 Berlin (DE)
(74) Vertreter: Müller, Wolfram Hubertus

(56) Entgegenhaltungen:
- WO-A2-2012/163937
- CN-A- 102 451 175
- WEGIEL LINDSAY A ET AL: "Phase Behavior of Resveratrol Solid Dispersions Upon Addition to Aqueous media", PHARMACEUTICAL RESEARCH, SPRINGER US, NEW YORK, vol. 32, no. 10, 15 May 2015 (2015-05-15), pages 3324 - 3337, XP035537496, ISSN: 0724-8741, [retrieved on 20150515], DOI: 10.1007/S11095-015-1709-Z
- AI L ET AL: "Solid dispersion for use in preparing grape wine or juice comprises resveratrol or polydatin, carrier, and additive", WPI / THOMSON,, vol. 2009, no. 15, 29 October 2008 (2008-10-29), XP002662558

## Beschreibung

Die Erfindung bezieht sich auf die Herstellung einer Zusammensetzung enthaltend Resveratrol und Curcumin.

Zum Hintergrund der Erfindung ist anzumerken, dass gemäß dem biopharmazeutischen Klassifikationssystem (BCS) Klasse IV-Verbindungen, die geringste orale Bioverfügbarkeit, sehr geringe Löslichkeit und sehr geringe intestinale Permeabilität unter allen pharmazeutischen Klassen von Medikamenten zeigen. Daher benötigen diese Medikamente ein kompatibleres und effizienteres Verabreichungssystem. Da ihre Löslichkeit in verschiedenen Medien eine Einschränkung darstellt, könnte sich die Beladung mit polymeren Arzneimitteln mit einem modifizierten Ansatz für sie als eine Lösung erweisen. Die Resorptionsrate von Arzneimitteln im Gastrointestinaltrakt (GI-T) wird durch eine Vielzahl von Faktoren beeinflusst, wie z.B. durch die physikalisch-chemische Natur, die Größe und das Molekulargewicht der Verbindungen, den Stoffwechsel, die physiologischen Funktionen, die Struktur und Oberfläche der Darmzellen usw.. Ungeachtet dieser Komplexität zeigt das von Amidon et al. und Lipinski et al. entwickelte Biopharmazeutische Klassifikationssystem (BCS) deutlich, dass die synthetisch gewonnenen Arzneistoffe, die durch die Einführung des Hochdurchsatz-Screenings (HTS) und der kombinatorischen Chemie in großem Umfang hergestellt wurden, auf der anderen Seite aber mit Herausforderungen durch schlecht wasserlösliche Arzneistoffe konfrontiert waren.

Basierend auf dem Biopharmazeutischen Klassifizierungssystem (BCS) werden die Medikamente je nach ihren Löslichkeits- und Permeabilitätseigenschaften in vier Kategorien eingeteilt, des Weiteren werden die Resorptionseigenschaften betrachtet:
- in Verbindungen der Klasse I, die eine hohe Löslichkeit, eine hohe Permeabilität und eine gute, nur durch die Geschwindigkeit der Magenentleerung kontrollierte Resorption aufweisen;
- in Verbindungen der Klasse II, die eine niedrige Löslichkeit, eine hohe Permeabilität und eine eingeschränkte, durch die Lösungsgeschwindigkeit des Wirkstoffes bestimmte Resorption aufweisen;
- in Verbindungen der Klasse III, die eine hohe Löslichkeit, eine niedrige Permeabilität und eine von den physikalisch Wirkstoffeigenschaften unabhängige Resorption aufweisen;
- in Verbindungen der Klasse IV, die eine niedrige Löslichkeit, eine niedrige Permeabilität und eine unbestimmte Resorption aufweisen.

Als dieses Klassifizierungssystem anschließend eingehend untersucht wurde, stellte sich heraus, dass die Arzneimittelformulierung und ihr Trägersystem gleichermaßen für die Bestimmung der Resorptionsrate und des Ausmaßes der Resorption im GI-T verantwortlich sind, wodurch die Bioverfügbarkeit und die therapeutische Wirkung der klassifizierten Arzneimittel erhöht werden. Es wurden ständig mehrere Ansätze zur Verbesserung der Wirkstoffabgabe durch erhöhte Löslichkeit und Permeabilität entwickelt und modifiziert, insbesondere für Verbindungen der Klassen II und IV. Die Ansätze wie Komplexierung, Mikronisierung, Kristallmodifikation, Erhöhung der Löslichkeit der Medikamente usw. sind zwar weiter erforscht, aber diese Techniken haben Einschränkungen, gerade vor allem, um die Löslichkeit und Permeabilität von Medikamenten der Klasse IV zu verbessern. Folglich wäre der beste Ansatz zur Verbesserung der Bioverfügbarkeit dieser Medikamente die Rückkehr in die Phase der Lead-Optimierung innerhalb der Medikamentenentwicklung und die Veränderung ihrer Strukturen, um die entsprechenden physikalisch-chemischen Eigenschaften zu erhalten, die eine höhere Löslichkeit und dadurch eine höhere Bioverfügbarkeit ermöglichen.

In den letzten Jahrzehnten wurden mehrere Arten von Festkörperdispersionen (SDs) entwickelt, die hinsichtlich des physikalischen Zustands des Wirkstoffs in der Matrix nicht alle gleich sind. Die gängigsten und attraktivsten Systeme sind amorphe feste Suspensionen oder Lösungen, bei denen die aktive Verbindung in amorpher Form bzw. molekular dispergiert ist. Der amorphe Zustand bzw. die molekulare Dispersion eines Wirkstoffs zeigt eine typisch höhere orale Bioverfügbarkeit im Vergleich zur kristallinen Form, da beide Verteilungen eine höhere freie Energie und eine bessere thermodynamische Aktivität aufweisen.

Amorphe SDs können durch verschiedene Herstellungsmethoden wie Lösungsmittelverdampfung, Sprühtrocknung, Schmelzen oder Heißschmelzextrusion (HME) hergestellt werden. In den verschiedenen Patenten/Patentanmeldungen werden dazu mehrere Methoden zur Herstellung von Formulierungen von lipophilen Wirkstoffen, darunter Resveratrol und Curcumin, mit Polymermatrizen vorgeschlagen, um eine bessere Löslichkeit in wässrigen Medien und damit eine höhere Bioverfügbarkeit zu erreichen:
So geht aus FR 2 758 459 eine fenofibrithaltige Zusammensetzung mit sofortiger Freisetzung sowie ein Verfahren zur Herstellung dieser Arzneizusammensetzung hervor, bei denen die gewünschte höhere Bioverfügbarkeit durch Pulverisieren des Wirkstoffes zu feinst zerkleinerter Form mit einer Größe kleiner als 20 µm erreicht werden soll. Das Verfahren umfasst die Herstellung einer Suspension des Fenofibrats, das zerkleinert ist bis zu einer Größe kleiner 20 µm, in einer Lösung eines hydrophilen Polymers, gegebenenfalls unter Verwendung eines oberflächenaktiven Mittels, und anschließend das Auftragen der Suspension auf einen hydrolöslichen Träger.

WO 2018/203294 beschreibt eine Zusammensetzung, bei der eine bessere Bioverfügbarkeit und Löslichkeit in einem wässrigen Medium ebenfalls durch Mikronisierung erreicht werden soll. Es wird eine flüssige pharmazeutische Zusammensetzung beschrieben, mit einer stabilen kolloidalen Dispersion, die polymere Partikel von Zein und β-Cyclodextrin in Suspension in einer hydroalkoholischen kontinuierlichen Phase umfasst, wobei die Partikel einen mittleren Durchmesser von weniger als 1 µm aufweisen, vorzugsweise im Bereich von 100 nm bis weniger als 1 µm, wobei die pharmazeutische Zusammensetzung mindestens einen in die Partikel eingearbeiteten Wirkstoff, wie Resveratrol und Curcumin, enthält.

Aus US 6,221,399 B1 geht ein Verfahren eines soliden Interpolymerkomplexes zur Verwendung als Matrix mit gesteuerter Freisetzung für eine orale Verabreichung hervor, bei dem die gesteuerte Freisetzung durch Herstellung fester Teilchen des Interpolymerkomplexes durch Sprühtrocknung bewirkt wird.

EP 2 581 089 B1 beschreibt ein Verfahren zur Herstellung einer Polyphenolzusammensetzung, umfassend einen Schritt, in dem ein kaum wasserlösliches Polyphenol und ein oder mehrere Vertreter methylierter Verbindungen von kaum wasserlöslichen Polyphenolen, wobei unter anderem Resveratrol und Curcumin als zu lösende Verbindungen eingesetzt werden, einer Wärmebehandlung bei 100°C bis 180°C in Gegenwart eines wässrigen Mediums ausgesetzt werden und in einem weiteren Schritt, die der Wärmebehandlung ausgesetzte Lösung auf 90°C oder weniger bei einer Kühlrate von 1°C/s oder mehr und 100°C/s oder weniger gekühlt wird.

Aus WO 2009/040818 A1 geht eine feste Zusammensetzung hervor, die bei Kontakt mit wässrigen Medien eine kolloidale Nanodispersion bildet, wobei die Zusammensetzung mindestens einen lipophilen Wirkstoff, wie Resveratrol, umfasst, der in engem Kontakt mit einer Polymermatrix aus einem amphiphilen und einem hydrophilen Polymer positioniert ist. Der lipophile Wirkstoff weist modifizierte physikalisch-chemische Eigenschaften auf, die entweder durch eine verminderte Schmelzenthalpie oder sowohl durch eine verminderte Enthalpie als auch eine verminderte Temperatur des Schmelzens dargestellt werden, im Vergleich zum gleichen losen anfänglich kristallinen lipophilen Wirkstoff, der als Ausgangspunkt für die Herstellung der Zusammensetzung verwendet wird.

Die CN 102 451 175 offenbart eine Resveratrol- und Bioflavonoidzusammensetzung. Die Wirkstoffe der Resveratrol- und Bioflavonoid-Zusammensetzung umfassen eine Resveratrol-Feststoffdispersion und eine Bioflavonoid-Feststoffdispersion in einem Gewichtsverhältnis von (1-10):(1-10).

Bislang waren alle derartigen Anstrengungen mittels bekannter SDs bezüglich Resveratrol und/oder Curcumin ohne hinreichenden Erfolg, insbesondere in medizinischer und wirtschaftlicher Hinsicht. Es konnte keine signifikant höhere Löslichkeit und keine signifikant höhere Bioverfügbarkeit von Resveratrol und/oder Curcumin im menschlichen Organismus erreicht und nachgewiesen werden.

Bezüglich Resveratrol belegt das Review " Resveratrol and its Human Matabolites - Effects on Matabolic Health and Obesity" von Margherita Springer und Sofia Moco, Nutriens 2019,11,143 , eindeutig die sehr geringe Bioverfügbarkeit von bis dahin bekannten und untersuchten verschiedenen Darreichungsformen von Resveratrol.

Als lösungsmittelfreies, einstufiges, kontinuierliches Verfahren bietet die Heißschmelzextrusion (HME) eine attraktive Alternative zu anderen Techniken, weshalb in den letzten 10-15 Jahren ein wachsendes Interesse an dieser Methode gezeigt wurde.

So schlägt WO 2019/159 174 vor, mittels HME eine feste Lösungszusammensetzung bereitzustellen, die ein oder mehrere Cannabinoide, einen oder mehrere nichtionische Emulgatoren und einen oder mehrere feste Matrixbildner umfasst, wobei das eine oder die mehreren Cannabinoide zusammen mit dem Emulgator und dem festen Matrixbildner in einem festen Lösungsmittelsystem gelöst sind.

Während eines HME-Prozesses schmilzt oder erweicht ein Material unter erhöhter/m Temperatur/Druck und wird mittels einer/zweier rotierenden Schnecke(n) durch eine Düse gepresst. Es gibt eine Vielzahl von nachgeschalteten Prozessen, die sogar kombiniert werden können, so dass eine Vielzahl von Dosierungsformen (z.B. Pellets, Tabletten, Granulate) gestaltet werden kann. Die durch die Schnecke(n) erzielte intensive Vermischung führt in der Regel zu einer gleichmäßigen Verteilung von Hilfsstoffen und Wirkstoffen. Die Freisetzungsrate und die Stabilität der Formulierung können je nach Wahl der Polymermatrix und der Additive maßgeschneidert werden. Der wichtigste in HME verwendete Hilfsstoff ist ein polymerer Träger auf Lipidbasis.

Die Auswahl eines geeigneten Polymers erfordert Kenntnisse über die physikalisch-chemischen Eigenschaften. Polymere, die in HME verwendet werden, sollten ein thermoplastisches Verhalten aufweisen, d.h. sie sollten bei der Verarbeitungstemperatur weich werden, ohne sich zu zersetzen, und sich beim Verlassen der Düse verfestigen.

Die Auswahl geeigneter Polymere und funktioneller Hilfsstoffe (z.B. Weichmacher, Antioxidationsmittel, Porenbildner) ist somit bei der Entwicklung amorpher SDs von großer Bedeutung.

Die thermische Stabilität ist eine erste Voraussetzung, jedoch sind auch andere Parameter von Bedeutung, um das Vermischen von Wirkstoffen und Hilfsstoffen und die thermodynamische Stabilität der endgültigen Darreichungsform zu gewährleisten. Physikalisch-chemische Eigenschaften, wie Löslichkeitsparameter, Glasübergangstemperatur, Schmelztemperatur, Hygroskopizität, wasserstoffbindende Donor- oder Akzeptorgruppen und mechanische Eigenschaften, sind allesamt Schlüsselparameter, die zur Erreichung der gewünschten Verbesserung der Löslichkeit, Bioverfügbarkeit und Stabilität beitragen.

Die Verwendung von lipidbasierten Hilfsstoffen kann ein Schlüssel für die Formulierung und Stabilisierung von lipophilen Verbindungen sein.

Aufgabe der Erfindung ist es, ein Verfahren zur Herstellung einer Zusammensetzung, die Resveratrol und Curcumin als natürliche lipophile Verbindungen enthält, bereitzustellen, das eine verbesserte Bioverfügbarkeit und Löslichkeit dieser Wirkstoffe bereitstellt.

Erfindungsgemäß wird die Aufgabe durch ein Verfahren mit den Merkmalen des Anspruch 1 gelöst.

Das Verfahre umfasst die Schritte des Zuführens der Bestandteile in einen Heißschmelzextruder, des Mischens und Erhitzens der Bestandteile bis zum Schmelzen und dass anschließende Extrudieren, Abkühlen und Formen, wobei alle diese Schritte zusammen einen einzigen kontinuierlichen Prozess bilden.

Resveratrol und Curcumin zusammen mit einem Emulgator oder den mehreren Emulgatoren und einem matrixbildenden Mittel oder mehreren matrixbildenden Mitteln in einem festen Lösungsmittelsystem löslich ausgebildet sind und die Zusammensetzung mittels Heißschmelzextraktion hergestellt ist.

Typischerweise hat die Zusammensetzung die Eigenschaft, dass sie nach Kontakt mit einer wässrigen Flüssigkeit, so zum Beispiel einer Körperflüssigkeit, eine Vielzahl von Teilchen freisetzt, wobei die Teilchen eine mittlere Teilchengröße von etwa 150 nm aufweisen und Resveratrol und Curcumin in den freigesetzten Teilchen im Wesentlichen gelöst sind.

Vorzugsweise ist das Resveratrol der Formel 3,5,4-trihydroxy-trans-stilben ausgewählt aus der Gruppe der Familien Dipterocarpaceae, Paeoniaceae, Vitaceae, Leguminosae, Gnetaceae, Cyperaceae, Polygonaceae Gramineae und Poaceae und das eine Curcumin die Formel (1E,6E)-1,7-Bis (4-hydroxy-3-methoxyphenyl) -1,6-heptadien-3,5-dione aufweist.

Gemäß einer weiteren Ausführungsform der Erfindung ist der mindestens eine nichtionische Emulgator ausgewählt aus der Gruppe, die aus Polysorbaten, Polysorbat 80, polyoxylhydriertem Rizinusöl, Saccharoseester, Saccharosedistearat, Tocopherylpolyethylenglykol 1000-Succinat, Sorbitanfettsäureester, Sorbitanmonooleat, Polyglycerylfettsäureester, Polyoxylglyceriden oder Salzen Derivaten oder Kombinationen davon besteht.

Es ist vorgesehen, dass der mindestens eine Emulgator ausgewählt ist aus nichtionischen oder anionischen Tensiden mit einem Hydrophilic-Lipophilic-Balance-Wert (HLB-Wert) von etwa 10 bis etwa 16 und ein zweiter Emulgator ausgewählt ist aus nichtionischen oder anionischen hydrophilen oder hydrophoben Tensiden mit einem HLB-Wert von etwa 4 bis etwa 12.

Weiter ist ein festes matrixbildendes Mittel vorgesehen ausgewählt aus der Gruppe bestehend aus Polyvinylcaprolactam-Polyvinylacetat-Polyethylenglykol-Pfropfcopolymer (z.B. Soluplus^{®} ), Polyvinylalkohol (PVA), vernetztem Copolymer aus Acrylsäure und einem hydrophoben C10-30-Alkylacrylat-Comonomer, Gelatine, Hydroxypropylmethylcellulose (z.B. Methocel), Methylcellulose, Hydroxypropylcellulose (Klucel), Hydroxyethylcellulose (z.B. Natrosol), Natriumcarboxymethylcellulose, Acrylat-Copolymere, Ammonio-Methacrylat-Copolymer Typ A oder B, Dimethylaminoethylmethacrylat-Butylmethacrylat-Methylmethacrylat-Copolymer (z.B. von Eudragit&trade), Methacrylsäure-Ethylacrylat-Copolymer, Polyvinylalkohol-Pfropfcopolymer (z.B. Kollicoat^{®} IR), Polyvinylacetat-Co-Crotonsäure, Polymethylmethacrylat und gepfropftes Polyethylenoxid, Lignine, Polyvinylpyrrolidon-Co-Vinylocetat (z.B. Kollidon VA64), Polyvinylpyrrolidon (z.B. Kollidon K90), Celluloseacetatphthalat, Hydroxypropylmethylcellulosephthalat, Carboxymethylethylcellulose, Hydroxypropylmethylcelluloseacetatsuccinat, Polyvinylacetat-Phtalat, Maltodextrin, Dextran, Polymethacrylsäure-Co-Ethylacrylat, Polymethacrylsäure-Co-Methylmethacrylat, Polymethacrylsäure-Co-Ethylacrylat, Polymethacrylsäure-Co-Methylmethacrylat, Polylactidsäure (PLA), Poly-L-Lactid (PLLA), Poly-D-Lactid (PDLA), Polylactid-Co-Glykolsäure (PLGA), Polyethylenoxid-Polypropylenoxid-Blockcopolymer (z.B. Poloxamer von Carbowax&trade), Polyethylenglykol (PEG1000, PEG1500,PEG 2000, PEG4000, PEG6000, PEG8000) und aus Mischungen davon.

Vorzugsweise ist ein zweites festes, matrixbildendes Mittel ausgewählt aus der Gruppe bestehend aus Bienenwachs, Carnaubawachs, Cetylpalmitat, Glycerylbehenat, Behensäure, Behenylalkohol, Glycerylmonostearat, Glycerylpalmitostearat, Glycerylstearat, hydriertes Rizinusöl, mikrokristallines Wachs, Paraffinwachs, Stearinsäure, Stearinsäurealkohol, Alkylsilikon, Silikonwachs, wachsartiges Polymethylsiloxan, PEG-Wachs oder Carbowax.

Es ist weiter vorgesehen, dass mindestens 80 % des Resveratrol und des Curcumins, gemessen durch Differential-Scanning-Kalorimetrie, in der festen Zusammensetzung vollständig gelöst.

Gemäß einer weiteren bevorzugten Ausführungsform beträgt das Verhältnis des Resveratrol und des Curcumins zu mindestens einem Emulgator von etwa 5:1 bis etwa 1:20 und beträgt das Verhältnis von a) dem Resveratrol und dem Curcumin zu b) der Mischung aus dem mindestens einen Emulgator und dem mindestens einem festen matrixbildenden Mittel etwa 2:1 bis etwa 1:20.

Das Gewichtsverhältnis von Resveratrol zu Curcumin liegt in Ausführungsformen im Bereich von 0,5 zu 1 bis 1 zu 2,5.

Die vorliegende Erfindung zeichnet sich durch den Vorzug aus, dass durch die Kombination von lipidbasierten Hilfsstoffen mit anorganischen Trägem extrudierte amorphe Festkörperdispersionen von Resveratrol in Kombination mit Curcumin als lipophile Modellverbindungen bereitgestellt werden können und diese Modellverbindungen eine besonders hohe Löslichkeit in wässrigen Medien und damit eine signifikant höhere Bioverfügbarkeit insbesondere auch wegen Verhinderung der üblichen, schnellen Metabolisierung aufweisen. Durch die signifikant erhöhte Freigabe der erfinderischen Zusammensetzung im menschlichen Darm wird zugleich die Aufnahme von Resveratrol und Curcumin durch die menschlichen Zellen gefördert. Die in diesen Zellen erfolgende Umwandlung in körpereigene Stoffe mit dem damit verbundenem Energiewechsel (Assimilation) führt letztlich zu einer signifikant höheren Bioverfügbarkeit der Wirkstoffe als bislang erreicht.

Die Erfindung weist insofern ein großes Potenzial auf, um die Wirksamkeit von Dosierungen vor allem bei Pharmaka zu verbessern. Aber auch für Lebensmitteltechnologien und insbesondere Nahrungsergänzungsmittel ergeben sich neue Möglichkeiten mit verbesserter Nachhaltigkeit.

Es spricht im Besonderen für die erfinderische Leistung, dass mittels der Heißschmelzextrusion aus Resveratrol in Kombination mit Curcumin amorphe Festkörperdispersionen ohne Zersetzung des polymeren inerten Trägers thermisch stabil hergestellt werden können, obwohl diese lipophilen Wirkstoffe signifikant unterschiedliche und hohe Schmelzpunkte (Resveratrol mit 253°C - 260°C und Curcumin mit 183°C) aufweisen.

Die Erfindung zeichnet sich darüber hinaus dadurch aus, dass die Herstellung therapeutisch einsetzbarer Mittel sowie von Naturstoffzusammensetzungen ermöglicht wird, in denen die synergistische Wirkung von Resveratrol und Curcumin nachhaltig und altersunabhängig belegt wird.

Resveratrol besitzt bekanntlich eine breite Palette biologischer Eigenschaften, darunter antioxidative, kardioprotektive, neuroprotektive, entzündungshemmende und krebsbekämpfende Wirkungen.

Kurkuma, ein Gewürz, das seit langem für seine medizinischen Eigenschaften anerkannt ist, hat sowohl in der medizinischen/wissenschaftlichen Welt als auch bei kulinarischen Enthusiasten Interesse gefunden, da es die Hauptquelle für das Polyphenol Curcumin ist. Es hilft bei der Behandlung von oxidativen und entzündlichen Zuständen, metabolischem Syndrom, Arthritis, Angstzuständen und Hyperlipidämie.

Die pflanzlichen Wirkstoffe, die in den Zusammensetzungen der vorliegenden Erfindung verwendet werden, sind standardisierte Kräuterextrakte. Es ist bedeutsam, dass diese Materialien, die zur Herstellung der erfindungsgemäßen Zusammensetzung verwendet werden, pflanzlichen Ursprungs sind. So wurde z.B. das resveratrolhaltige Pflanzenmaterial auf dem Feld geerntet, Resveratrol wurde in Rotweinen und in verschiedenen anderen menschlichen Nahrungsmitteln gefunden.

Bis heute wurden 92 neue Resveratrol-Verbindungen, darunter 39 Dimere, 23 Trimere, 13 Tetramere, 6 Resveratrol-Monomere, 6 Hexamere, 4 Pentamere und 1 Oktamer aus den Familien Dipterocarpaceae, Paeoniaceae, Vitaceae, Leguminosae, Gnetaceae, Cyperaceae, Polygonaceae Gramineae und Poaceae gemeldet. Von diesen Familien machen die Dipterocarpaceae, die 50 Resveratrole enthalten, die Mehrheit aus, wobei 7 Dipterocarpaceae-Gattungen beteiligt sind, darunter Vatica, Vateria, Shorea, Hopea, Neobalanocarpus, Dipterocarpus und Dryobalanops.

Kurkuma, eine rhizomatöse, krautige, mehrjährige Pflanze (Curcuma longa) aus der Familie der Ingwergewächse wird in verschiedenen, dem Fachmann gut bekannten Formen gewonnen, darunter als getrocknetes Pflanzenmaterial aus Wurzeln, Blättern, Sprossen, Blüten und ganzen Pflanzen sowie als wässrige und nichtwässrige Extrakte und Öle.

Wie erläutert betrifft die Erfindung die Herstellung einer Zusammensetzung mittels des Heißschmelzextrusionsverfahrens (HME).

Von besonderem Interesse ist in diesem Zusammenhang das Heißschmelzmischen, bei dem statt eines Einschneckenextruders ein Doppelschneckenextruder verwendet wird, um die therapeutische Verbindung mit einem inerten Träger noch effizienter zu mischen, um eine feste Dispersion zu bilden. Typischerweise werden die Arbeitsbereiche eines Einschnecken- und Doppelschneckenextruders beheizt, um das Mischen der therapeutischen Verbindung mit dem Träger zu beschleunigen.

Nach dem Stand der Technik ist in einigen Fällen die Erwärmung der Zone des Heißschmelzextruders auf eine Temperatur über dem Schmelzpunkt der therapeutischen Verbindung ungeeignet, da diese Temperatur die Schmelztemperatur des inerten Trägers übersteigen kann und dieser sich dadurch zersetzt. Darüber hinaus zersetzen sich nach dem Stand der Technik einige therapeutische Verbindungen beim Heißschmelzen selbst.

Es bestand daher die Notwendigkeit, ein Verfahren bereitzustellen, welches es ermöglicht, auch therapeutische Verbindungen mit einem hohen Schmelzpunkt von hochkristallin auf amorph zu übertragen und die sich beim nahezu Erreichen des Schmelzpunktes nicht zersetzen. Die vorliegende Erfindung wird diesen Anforderungen durch Verwendung eines Lösungsvermittlers gerecht. Geeignete Lösungsvermittler sind Blockcopolymere, zum Beispiel nichtionische synthetische Blockcopolymere aus Ethylenoxid und Propylenoxid, wie Poloxomer 407, insbesondere F127. In einer anderen Ausführungsform umfassen Lösungsvermittler neben der bereits genannten Klasse der Blockcopolymere auch andere Tenside.

Der spezifizierte Lösungsvermittler erlaubt es, die Verarbeitungstemperatur des therapeutischen Präparates zu senken, um dessen Integrität zu erhalten und gleichzeitig den Übergang des physikalischen Zustands von kristallin und/oder thermisch labil zu amorph zu gewährleisten. Darüber hinaus ermöglicht das Verfahren für die erfindungsgemäßen Zusammensetzungen Träger oder Polymere zu verwenden, die sich normalerweise bei hohen Temperaturen zersetzen, wodurch zugleich eine höhere Vielseitigkeit der Zusammensetzungen gewährleistet wird.

Mit dem folgenden Ausführungsbeispiel soll die Erfindung verdeutlicht werden.

Zum Einsatz kommen die in der nachfolgenden tabellarischen Übersicht aufgeführten Substanzen mit den genannten Anteilen.

Die angegebenen Mengen stellen den Mittelwert aus zwei Versuchsreihen dar.

### Ausführungs-Beispiel:

Übersicht zu den Ausgangssubstanzen der durch das Heißschmelzverfahren hergestellten Zusammensetzung

| **Gew..%** | **Substanz** | **Menge (gr)** |
|---|---|---|
| 16,67 | Resveratrol; 99,84 | 200,00 |
| 17,50 | Curcumin,; 95,5 | 210,00 |
| 3,33 | Polyethylenglycol 2000 | 40,00 |
| 30,00 | Poloxamer 407 | 360,00 |
| 10,00 | ß-Cyclodextrin | 120,00 |
| 4,17 | Vitamin E TPGS | 50,00 |
| 1,67 | Compritol 888 ATO | 20,00 |
| 2,50 | Aerosil 200 | 30,00 |
| 14,17 | Polyvinylalkohol PVA | 170,00 |
| **100,00** | | **1.200,00** |

- Resveratrol,: mit einer Reinheit von 99,84 %,
- Curcumin,: mit einer Reinheit von 95,5 %;
- Polyethylenglykol 2000,: als hydrophiles nichtionisches Tensid;
- Poloxamer 407,: als hydrophiles nichtionisches Tensid,
- β-Cyclodextrin,: als Mittel, das mit einem hydrophoben Inneren und einem hydrophilen Äußeren Komplexe mit hydrophoben Verbindungen bildet und einem pharmakologischen Präparat Löslichkeit und Stabilität verleiht; Einschlussverbindungen von Cyclodextrinen mit hydrophoben Molekülen sind in der Lage, in Körpergewebe einzudringen und können deshalb verwendet werden, umbiologisch aktive Verbindungen unter bestimmten Bedingungen und an bestimmten Orten freizusetzen;
- Vitamin E TPGS,: als eine mit Wasser mischbare Form von Vitamin E, bestehend aus einem hydrophoben Vitamin E-Teil und einer hydrophilen PEG-Kette;
- Compritol 888 ATO,: als Mittel zur Bildung einer Lipidbarriere bei der Heißschmelzextrusion und als Träger für den Schutz empfindlicher Wirkstoffe und für Nanopartikel-Technologien zur Verbesserung der Wirkstoffverträglichkeit;
- Aerosil 200,: als Mittel zur Beeinflussung der Rheologie und zu Thixotropiekontrolle sowie als Antiabsetz-, Verdickungs- und Antiabsackmittel und zur Verbesserung des freien Flusses;
- Polyvinylalkohol PVA,: als Mittel zur Verbesserung der Löslichkeit aktiver Inhaltsstoffe von Extrudaten und der Stabilität von Extrudatmaterialien.

Die genannten Substanzen wurden in einer Charge von 1.200 g vor dem Extrusionsprozess für 10 Minuten gemischt. Zum Einsatz kam ein Mischer vom Typ Turbula TF 2 (Schweiz). Das so vorgemischte Pulver wurde anschließend in den Trichter des Extruders gefüllt- Die Extrusion erfolgte dann in einem Einschnecken-Labor-Heißschmelzextruder (Thermo Fischer HME PolyLab-OS, Deutschland), der mit einer nitrierten Einschnecke aus Edelstahl DIN 1.8550 mit einem Durchmesser von 19,05 mm (3/4") ausgestattet ist.

Der Extrusionsprozess wurde bei 80 U/min und einem Vorschub von 1 kg/h durchgeführt und die Temperatur dabei mit 105 ± 1°C gehalten.

Im Gegensatz zu Pellets, die durch Nassgranulation gewonnen werden, benötigt das Heißschmelzextrusionsverfahren der vorliegenden Erfindung keine Granulierungsflüssigkeit, wie z.B. Wasser, Methanol, Ethanol, Isopropanol oder Aceton.

Das so gewonnene Extrudat wurde zu einem Granulat zerkleinert, das die innere Phase der pharmazeutischen Zusammensetzung bildet. Die erforderliche Granulatgröße für eine bestimmte pharmazeutische Zusammensetzung, die entwickelt wird, ist von einem Fachmann zu bestimmen. Eine geeignete Partikelgröße ist beispielsweise kleiner oder gleich 1000 Mikron, 750 Mikron, 500 Mikron oder 250 Mikron.

In einer weiteren Charge gleicher Zusammensetzung der Ausgangsmaterialien, wie in der Tabelle des Ausführungs-Beispiels aufgeführt, wurde bei einer Extrusionstemperatur von 130°C das Extrudat direkt zu Tabletten geformt, zu Mikropartikeln zerkleinert oder in eine andere Form verarbeitet, wie es einem Fachmann bekannt ist.

Das Granulat wurde in Form von Partikeln eines eingekapselten therapeutischen Wirkstoffs oder einer Schicht mit kontrollierter Freisetzung als Retardierungsmittel erhalten. Die resultierenden Granulate sind Partikel eines therapeutischen Wirkstoffs, die mit einer körnigen Exzipientenschicht überzogen oder im Wesentlichen umhüllt sind, oder, in einer anderen Ausführungsform, Partikel eines therapeutischen Wirkstoffs, die in einem körnigen Exzipienten eingekapselt oder im Wesentlichen eingekapselt sind.

Nach der Gewinnung der Granulate wurden sie zu oralen Formen verarbeitet, so zu festen oralen Darreichungsformen wie Tabletten, Pillen, Lutschtabletten, Mikrotabletten, Kapseln oder Sachets, wobei zusätzliche Standard-Trägerstoffe hinzugefügt wurden, die die äußere Phase der pharmazeutischen Zusammensetzung bilden. Die äußere Phase der pharmazeutischen Zusammensetzung kann auch einen zusätzlichen therapeutischen Wirkstoff enthalten. Zu diesen festen oralen Darreichungsformen gehören z,B. orale Standarddarreichungsformen.

Beispiele für diese Standard-Trägerstoffe sind unter anderem Freisetzungsverzögerer, Weichmacher, Sprengmittel, Bindemittel, Schmiermittel, Gleitmittel, Stabilisatoren, Füllstoffe und Verdünnungsmittel. Um der festen oralen Form bestimmte orale Eigenschaften zu verleihen, kann ein geübter Anwender mit einfachen Standardexperimenten leicht einen oder mehrere der oben genannten Hilfsstoffe auswählen. Die Menge jedes verwendeten Hilfsstoffs wird in einem üblichen Bereich variiert,

Beispiele für pharmazeutisch akzeptable Sprengmittel sind unter anderem Stärke, Tone, Cellulosen, Alginate, Gummis, vernetzte Polymere, z.B. vernetztes Polyvinylpyrrolidon oder Crospovidon (z. B. Polyplasdone XL von Ashland Inc.), vemetztes Natriumsalz von Kohlenhydrat oder Kohlenhydrat Croscarmellose (z.B. AC-DI-SOL von FMC), sowie das vernetzte Calciumsalz von Carboxymethylcellulose, Soja-Polysacchariden und Guarkernmehl. Die Menge des Sprengmittels liegt zwischen etwa 0 Gew.-% und etwa 10 Gew.-%, bezogen auf das Gewicht der Zusammensetzung. In einer Verkörperung beträgt die Menge des Sprengmittels etwa 0,1 Gew.-% bis etwa 1,5 Gew.-%, bezogen auf das Gewicht der Zusammensetzung.

Beispiele für pharmazeutisch akzeptable Bindemittel sind unter anderem Stärken, Cellulosen und deren Derivate, z.B. mikrokristalline Cellulose (z.B. Avicel PH von FMC), Hydroxypropylcellulose, Hydroxyethylcellulose und Hydroxypropylmethylcellulose (z.B. Methocel von Dow Chemical Corp.), Saccharose, Dextrose, Maissirup, Polysaccharide und Gelatine. Die Menge des Bindemittels liegt zwischen etwa 0 Gew.-% und etwa 50 Gew.-%, zum Beispiel 10-40 Gew.-%, bezogen auf das Gewicht der Zusammensetzung.

Beispiele für pharmazeutisch akzeptable Schmiermittel und pharmazeutisch akzeptable Gleitmittel sind u.a. kolloidales Siliziumdioxid, Magnesiumtrisilikat, Stärke, Talkum, Trikalziumphosphat, Magnesiumstearat, Aluminiumstearat, Kalziumstearat, Magnesiumcarbonat, Magnesiumoxid, Cellulose und Cellulose-Cellulose. Die Menge der Schlichte liegt zwischen etwa 0 Gew.-% und etwa 10 Gew.-%, bezogen auf das Gewicht der Zusammensetzung. In einer Ausführung beträgt die Menge der Schlichte von etwa 0,1 Gew.-% bis etwa 1,5 Gew.-%, bezogen auf das Gewicht der Zusammensetzung. Die Menge des Gleitmittels beträgt von etwa 0,1 Gew.-% bis etwa 10 Gew.-%.

Beispiele für pharmazeutisch annehmbare Hilfsstoffe und pharmazeutisch annehmbare Verdünnungsmittel sind unter anderem Puderzucker, Presszucker, Dextrate, Dextrin, Dextrose, Laktose, Mannit, mikrokristalline Cellulose, Pulvercellulose, Sorbit, Saccharose und Talkum. Die Menge des Füllstoffs und/oder Verdünnungsmittels beträgt z.B. von etwa 15 Gew.-% bis etwa 40 Gew.-%, bezogen auf das Gewicht der Zusammensetzung.

Im Heißschmelzextruder wird die Mischung auf Temperaturen (s) unterhalb des Schmelzpunkts des therapeutischen Präparats und des Schmelzpunkts des Lösungsvermittlers erhitzt. Während des Erhitzens wird die Mischung mit der/(den) Schnecke(n) des Heißschmelzextruders gemischt. Die Mischung wurde bei erhöhter Temperatur gehalten und für eine ausreichende Zeit gerührt, um ein körniges Produkt zu bilden. Nachdem die Mischung über die gesamte Länge des Zylinders geleitet wurde, wird ein körniges Produkt, das ein Extrudat ist, erhalten und die körnige Mischung wird gekühlt.

Unter dem Begriff "therapeutische Verbindung" ist in diesem Zusammenhang eine Verbindung zu verstehen, die als Bestandteil einer pharmazeutischen Zusammensetzung geeignet ist, zur Verabreichung an Menschen, beispielsweise zur Vorbeugung, Behandlung oder Verringerung von Symptomen einer bestimmten Krankheit oder eines bestimmten Umstandes, unter dem der Mensch leidet.

Der Begriff "pharmazeutisch akzeptabel" bezieht sich auf Verbindungen, Materialien, Zusammensetzungen und/oder Dosierungsformen, die nach den medizinischen Anforderungen für den Kontakt mit menschlichem Gewebe geeignet sind, ohne übermäßige Toxizität, Reizung, allergische Reaktion und andere Komplikationen in einem angemessenen Nutzen/Risiko-Verhältnis.

In diesem Zusammenhang bedeutet der Begriff "therapeutische Verbindung" jede Verbindung, Substanz, jedes Medikament, jede Droge oder jeder Wirkstoff, die eine therapeutische oder pharmakologische Wirkung hat und die für die Verabreichung an einen Menschen geeignet ist, in Form einer Zusammensetzung, die in erster Linie für die orale Verabreichung geeignet ist.

Der Begriff "schwer löslich" bezieht sich auf Verbindungen mit geringer oder sehr geringer Löslichkeit, wie sie im US-Arzneibuch definiert sind, z.B. wenn ein Teil der aufzulösenden Verbindung etwa 100 bis 10.000 Teile Lösungsmittel benötigt.

Der Begriff "kristallin" oder "kristalline Form" bezeichnet in diesem Zusammenhang einen physikalischen Zustand, der durch eine geordnete dreidimensionale Anordnung von Atomen, Ionen, Molekülen oder molekularen Assoziationen gekennzeichnet ist. Kristallformen sind durch das Vorhandensein eines Kristallgitters aus asymmetrischen Einheiten gekennzeichnet, die sich in wohldefinierter Symmetrie in den sich im dreidimensionalen Raum wiederholenden Gitterzellen befinden. Im Gegensatz zu dem angegebenen Begriff bezieht sich der Begriff "amorphe" oder "amorphe Form" auf eine unorganisierte (ungeordnete) Struktur.

Der physikalische Zustand der therapeutischen Verbindung wird mit Standardanalysemethoden wie Röntgenbeugung, Polarisationslichtmikroskopie und/oder Differential-Scanning-Kalorimetrie bestimmt.

Wie hier verwendet, bezieht sich der Begriff "thermisch labiler therapeutischer" Wirkstoff auf einen therapeutischen Wirkstoff, bei welchem ein spontaner Abbau oder eine spontane Zersetzung erfolgt, wenn der therapeutische Wirkstoff über oder annähernd auf seinen Schmelzpunkt erhitzt wird.

In diesem Zusammenhang bezieht sich der Begriff "hoher Schmelzpunkt" auf den Schmelzpunkt oder die niedrigste Temperatur im Bereich des Schmelzpunktes, der bei Resveratrol größer oder gleich 253°C und bei Curcumin 183°C ist.

Beispiele für therapeutische Klassen von therapeutischen Verbindungen sind unter anderem Antazida, entzündungshemmende Medikamente, Koronardilatatoren, Hirndilatatoren, periphere Vasodilatatoren, Antiinfektiva, Psychopharmaka, Antimanika, Stimulanzien, Antihistaminika, Krebstherapeutika, Abführmittel, abschwellende Mittel, Vitamine, enterische Beruhigungsmittel, Antidiarrhoika, antiangiöse therapeutische Präparate, gefäßerweiternde, antiarrhythmische, blutdrucksenkende therapeutische Präparate, gefäßverengende Mittel und Mittel zur Behandlung von Migräne, Antikoagulantien und antithrombotische therapeutische Präparate, Analgetika, Antipyretika, Hypnotika, Beruhigungsmittel, Antiemetika, Medikamente gegen Übelkeit, Antikonvulsiva, neuromuskuläre therapeutische Verbindungen, hyper- und hypoglykämische Mittel, schilddrüsenstimulierende und antithyreotische Medikamente, Diuretika, Antispasmodika, Uterusrelaxantien, Mineral- und Lebensmittelzusätze, Anti-Adipositas-Therapeutika, anabolische Therapeutika, erythropoetische Therapeutika, antiasthmatische Therapeutika, Expektoranzien, Antitussiva, Mukolytika, Therapeutika gegen Harnvergiftung sowie orale oder lokale Substanzen, Anti-Adipositas-Therapeutika, anabolische Therapeutika, erythropoetische Therapeutika, antiasthmatische Therapeutika, Expektorans, Therapeutika gegen Harnvergiftung sowie therapeutische Verbindungen oder topische Mittel in der Mundhöhle sowie Antitussiva, Mukolytika.

Die therapeutischen Verbindungen sind in den pharmazeutischen Zusammensetzungen der vorliegenden Erfindung in einer therapeutisch wirksamen Menge oder Konzentration enthalten. Die angegebene therapeutisch wirksame Menge oder Konzentration ist einem Fachmann bekannt und variiert je nach verwendetem Therapeutikum und der angegebenen Indikation. Beispielsweise liegt die Menge der therapeutischen Verbindungen nach der vorliegenden Erfindung zwischen etwa 0,05 Gew.-% und etwa 99 Gew.-%, bezogen auf das Gewicht der pharmazeutischen Zusammensetzung. In einer Ausführung beträgt die Menge der therapeutischen Verbindung zwischen etwa 10 Gew.-% bis etwa 95 Gew.-%, bezogen auf das Gewicht der pharmazeutischen Zusammensetzung.

Wie hier verwendet, bezieht sich der Begriff "Träger" auf eine pharmazeutisch akzeptable Matrix, die zur Herstellung einer festen oder molekularen Dispersion einer therapeutischen Verbindung geeignet ist. Geeignete Träger sind in erster Linie Polymere oder Copolymere oder Mischungen davon. Zu den Arten von Polymeren gehören unter anderem wasserlösliche, wasserquellbare und wasserunlösliche Polymere sowie Kombinationen davon.

Ein Beispiel für ein geeignetes Blockcopolymer ist das Poloxamer 188, das unter dem Handelsnamen Pluronic F68 verkauft wird.

### Weitere Beispiele für Polymere sind u.a. aber nicht ausschließlich;

Homopolymere und Copolymere von N-Vinyllactamen, Homopolymere und Copolymere von N-Vinylpyrrolidon (z.B. Polyvinylpyrrolidon), Copolymere aus N-Vinylpyrrolidon und Vinylacetat oder Vinylpropionat, hochmolekulare Polyalkylenoxide wie Polyethylenoxid und Polypropylenoxid sowie Copolymere aus Ethylenoxid und Propylenoxid, Vinylacetat-Polymere, wie z.B. Copolymere aus Vinylacetat und Crotonsäure, insbesondere hydrolysiertes Polyvinylacetat, Polyvinylalkohol und Oligo- und Polysaccharide, wie Carrageenane, Galaktomannane und Xanthan oder Mischungen aus einer oder mehreren dieser Verbindungen.

Geeignete Träger sind vor allem Verbindungen, die sich durch niedrige Glasübergangstemperaturen (Tg) auszeichnen. Beispiele für Träger mit niedrigen Tg sind unter anderem PVP K30, PVP K17 und PVP /VA.

Neben dem Polymer kann der Träger andere pharmazeutisch akzeptable Inhaltsstoffe, z.B, Weichmacher, enthalten.

In diesem Zusammenhang bezieht sich der Begriff "Weichmacher" auf ein Material, das in der pharmazeutischen Zusammensetzung enthalten ist, um die Tg und die Viskosität der Polymerschmelze durch Vergrößerung des freien Volumens zwischen den Polymerketten zu verringern.

Zu den Weichmachern gehören z.B. aber nicht ausschließlich:
- Wasser, Zitronensäureester (z.B. Triethylcitrat, Triacetin), niedermolekulare Polyalkylenoxide (z.B. Polyethylenglykole, Polypropylenglykole, Polyethylen-/Propylenglykole), Glycerin, Pentaerythrit, Monoacetat, Diacetat, Triacetat oder Natriumdiethylsulfosuccinat und
- therapeutische Verbindungen in freier Form

Die Konzentration des Weichmachers liegt zwischen etwa 0 Gew.-% und 15 Gew.-%, zum Beispiel zwischen 0,5 Gew.-% bis 5 Gew.-%, bezogen auf das Gewicht der pharmazeutischen Zusammensetzung. Beispiele für Weichmacher sind auch im Handbook of Pharmaceutical Additives, Ash et al., Gower Publishing (2000 ), enthalten.

Wie hier verwendet, bezieht sich der Begriff "Lösungsvermittler" auf ein Material, das in der Lage ist, eine therapeutische Verbindung und/oder ein Polymer zu lösen oder teilweise zu lösen. Als Lösungsvermittler sind Tenside besonders geeignet.

Der in diesem Zusammenhang verwendete Begriff "Tensid" umfasst nichtionische Tenside, anionische Tenside usw. sowie geeignete Kombinationen von zwei oder mehrerer dieser Tenside bekannter Art.

Ein Beispiel für ein geeignetes Tensid ist zunächst das Poloxamer 407 (z.B. Pluronic F127 von BASF oder Synperonic PE/F 127von Croda). Der Einsatz eines Lösungsvermittlers erfolgt gegebenenfalls zusammen mit einem Weichmacher und einem Träger.

Die Menge des Lösungsvermittlers beträgt etwa 5 Gew.-% bis etwa 40 Gew.-% bezogen auf das Gesamtgewicht der Extrudatzusammensetzung.

In ähnlicher Weise beträgt die Menge des therapeutischen Präparates etwa 0,01 Gew.-% bis etwa 50 Gew.-% bezogen auf das Gewicht der Extrudatzusammensetzung und die Menge des Trägers etwa 1 Gew.-% bis etwa 99 Gew.-% bezogen auf das Gewicht der Zusammensetzung.

Der Begriff "Granulierung aus der Schmelze" bezeichnet in diesem Zusammenhang eine typische Methode zur Gewinnung einer molekularen Dispersion des ursprünglichen hochkristallinen und/oder thermisch labilen Therapeutikums mittels Einsatzes eines Heißschmelzextruders.

Der Heißschmelzextruder besteht in der Regel aus einer oder mehreren rotierenden Schnecke(n) in einem feststehenden Zylinder mit einer Düse und einem optionalen Formkopf, der sich an einem Ende des Zylinders befindet. Durch die Rotation der Schnecke(n) im Zylinder wird über die gesamte Länge der Schnecke(n) eine gleichmäßig verteilte Mischung der Materialien (z.B. therapeutischer Wirkstoff, Trennmittel und andere notwendige Hilfsstoffe) erreicht.

Konventionell umfasst ein Heißschmelzextruder drei Zonen: eine Beschickungszone, eine Heizzone und eine Dosierzone. In der Beschickungszone werden die Rohstoffe z.B. mittels eines Trichters in den Extruder eingeführt. Die Rohstoffe werden ohne Lösungsmittel direkt in den Trichter gefüllt. In der Heizzone werden die unbehandelten Bestandteile auf die gewünschte Verarbeitungstemperatur erhitzt. Die Verarbeitungstemperatur übersteigt nicht die Zersetzungstemperatur der Materialien. So beträgt beispielsweise die Abbautemperatur von Poloxamer 407 175°C. Die Verarbeitungstemperatur wird daher im Bereich von ca. 50°C bis unter 175°C variiert, beispielsweise von 150°C bis ca. 170°C. Nach der Heizzone folgt eine Dosierzone, in der die zu mischenden Materialien optional durch einen Extruder-Formkopf geführt werden, um eine spezifische Materialform zu erhalten. Zu den Heißschmelzextrudertypen, die im erfindungsgemäßen Verfahren eingesetzt werden, gehören Ein- und Doppelschneckenextruder.

In einem weiteren Beispiel wurde die Formulierung mit den Substanzen gemäß Ausführungs-Beispiel zunächst 1 Minute gemischt und die so entstandene Mischung dann in die Beschickungszone oder in den Trichter eines Doppelschneckenextruders überführt. Ein dafür geeigneter Doppelschneckenextruder ist der Thermo Haake PolyLab Extruder Nr. 567-2020 der Thermo Fisher Scientific GmbH, Karlsruhe, Deutschland.) Der angegebene Heißschmelzxtruder verfügt über eine Mischzone. Der Heißschmelzxtruder wurde auf eine Temperatur von 150°C erhitzt. Das Material durchläuft den Heißschmelzxtruder für ca. 2 Minuten.

Ein halbfestes Extrudat wurde bei einer Temperatur von ca. 100°C erhalten. Um das Extrudat schnell auszuhärten, wurde es in einen Kühlschrank gelegt. Das Extrudat kann jedoch auch durch Luftkühlung ausgehärtet werden. Anschließend wurde das Extrudat zerkleinert.

Die nach dem erfindungsgemäßen Verfahren erhaltene fertige trockene, körnige Zusammensetzung weist einen, zwei oder mehr lipophile Wirkstoffe auf, die ein Polymer mit den Eigenschaften einer hydrophil-hydrophoben Matrix enthält. Diese Matrix wird so erhalten, dass der lipophile Wirkstoff eine reduzierte Schmelzenthalpie oder zumindest eine Verminderung sowohl der Schmelzenthalpie als auch des Schmelzpunktes des lipophilen Wirkstoffes im Vergleich zu dem als Ausgangsmaterial für die Herstellung der Zusammensetzung verwendeten lipophilen Hauptwirkstoff aufweist.

Die mit der erfindungsgemäßen Zusammensetzung durch die Heißschmelzextrusion erreichte hohe Löslichkeit im Sinne der Freisetzung von Resveratrol und Curcumin wurde in einer Studie in einem standardisierten Microemulsions-System, hier SMEDDS (selfmicroemulsifying drug delivery system) in vitro mittels Hochleistungsflüssigkeitschromatographie (HPLC) untersucht und nachgewiesen. Dazu wurden die Darmbedingungen eines hungrigen und eines gesättigten Menschen simuliert. Zum Einsatz kam eine extrudierte pharmazeutische Zusammensetzung, hergestellt aus den Substanzen gemäß dem Ausführungs-Beispiel.

Die Freisetzung (Löslichkeit) des Extrudats mit Resveratrol und Curcumin wurde mit unterschiedlichen Dispersionen, enthaltend rohes Resveratrol bzw. rohes Curcumin, verglichen. Verwendet wurde dafür rohes Resveratrol (aus Japan bezogen, Lot # 093016TR) mit einer Reinheit von 99% und rohes Curcumin-Ionga-Puiver mit einer Reinheit von 95% (bezogen aus Indien LOT # AB88703). Das rohe Resveratrol und das rohe Curcumin wurden dazu in Acetonitril von HPLC-Qualität, Eisessig und Wasser (bezogen von J.T. Baker, Deutschland) gegeben.

Eine Standard-Stammlösung wurde durch Auflösen von 20 mg rohes Resveratrol und 20 mg rohes Curcumin in 20 ml Acetonitril hergestellt (die Konzentration betrug somit etwa 1,0 mg/ml).

Für die zu prüfende Probe wurden 25 mg der erfindungsgemäß extrudiertenen Zusammensetzung in einen 25 ml Messkolben gegeben und anschließend mit Verdünnungsmittel auf das Volumen von 25 ml verdünnt und dabei gemischt.

Die HPLC-Analyse wurde auf HPLC-Systemen von Summit Dionex (Deutschland) mit Photodiodenarray (PDA) und UV-VIS-Detektoren und Chromeleon Version 6.80-Softwarepaketen durchgeführt.

Als Säule wurde eine solche der Dr.Maisch GmbH, Reprosil-Pur, C-18-AQ, 5µ, 250×4,6 mm mit Phenomenex Security Guard C18 4x3,0 mm genutzt.

Zusammensetzung der mobilen Phase: 500ml Wasser, 500ml Acetonitril und 20ml
- Essigsäure-Eiswasser;
- Temperatur der Säule: 35°C;
- Flussrate: 1,0 ml/min;
- Detektionswellenlänge: 306 nm für Resveratrol; 425 nm für Curcumin;
- Injektionsvolumen: 5 µl.
- Laufzeit: 15 min;
- Verdünnungsmittel: 50% Acetonitril und 50% Wasser;
- RT von Resveratrol: etwa 3,5 min;
- RT von Curcumin: etwa 9 min.

Für die invitro-Freisetzung von Resveratrol und Curcumin nach SMEDDS wurde jeweils eine 1.000 mi-Lösung mit FaSSIF, mit FeSSIF und mit 0,0175 molare Natrium-Laurylsulfat-Lösung als Lösungsmedium unter Rühren bei 100 U/min und 37° C verwendet.

Gemäß den Vorgaben des standardisierten Testverfahrens SMEDDS werden die Darmbedingungen eines hungrigen Menschen mittels einer Verdauungsflüssigkeit "FaSSIF" und die Darmbedingungen eines gesättigten Menschen mittels einer Verdauungsflüssigkeit "FeSSIF' simuliert, wobei hier beide Flüssigkeiten aus FaSSIF/FeSSIF-Pulver der Fa. Biorelevant.com Ltd., London, hergestellt wurden.

Des Weiteren wurde gemäß SMEDDS-Vorgaben zum Vergleich 1.000 ml 0,0175 molare anionische Natrium-Laurylsulfat-Lösung (SLS) als Tensid für das Medium verwendet.

Proben von je 5 ml wurden nach 0, 15; 30, 45 und 120 Minuten entnommen. Gleichzeitig wurde ein äquivalentes Volumen von je 5 ml frisches Lösungsmedium hinzugefügt, um das entnommene Volumen zu kompensieren. Die Probe wurde durch einen 0,45 µm-Filter filtriert und die Konzentration von Resveratrol und Curcumin mittels HPLC-Analyse festgestellt. Die Untersuchung erfolgte gemäß international anerkannten USP-Standards. Diese lassen eine maximal zulässige 5 % ige Abweichung in beiden Richtungen zu.

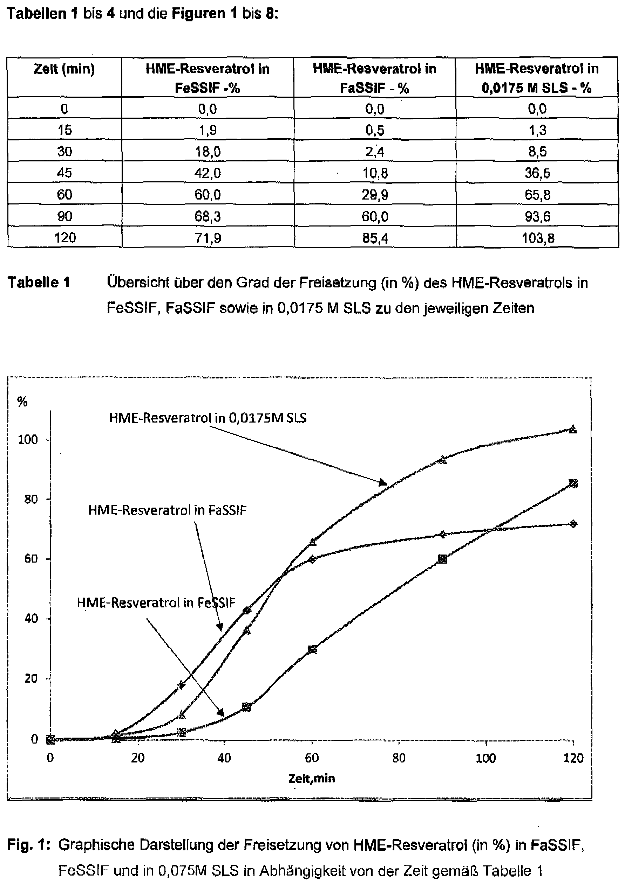

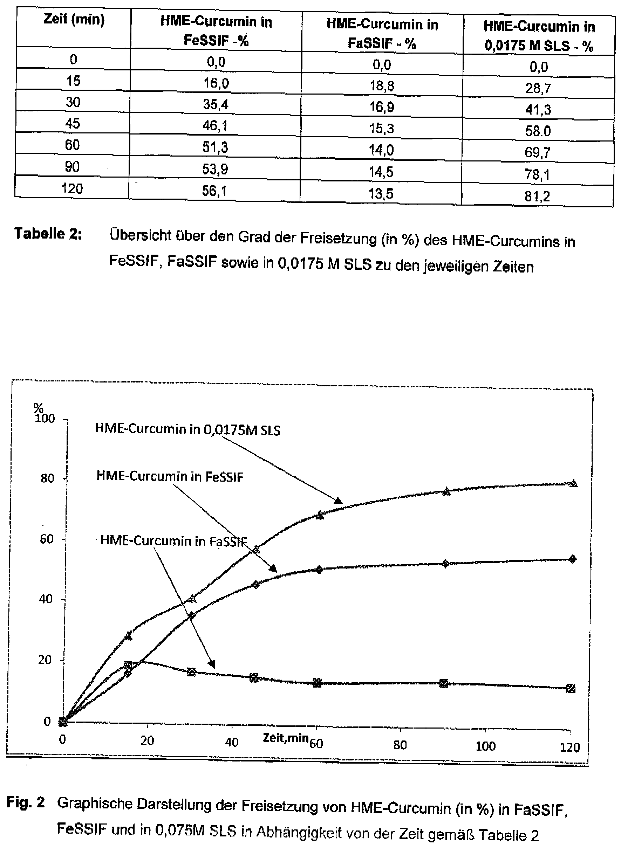

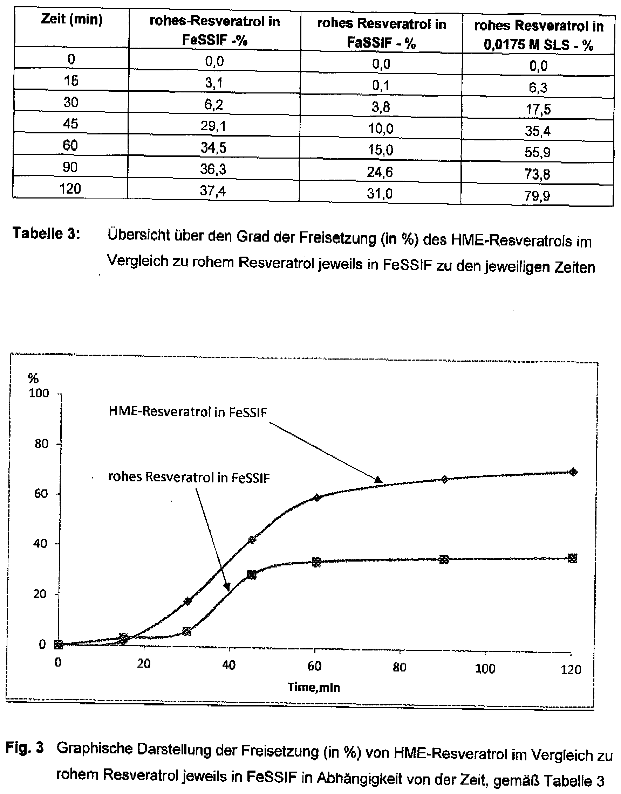

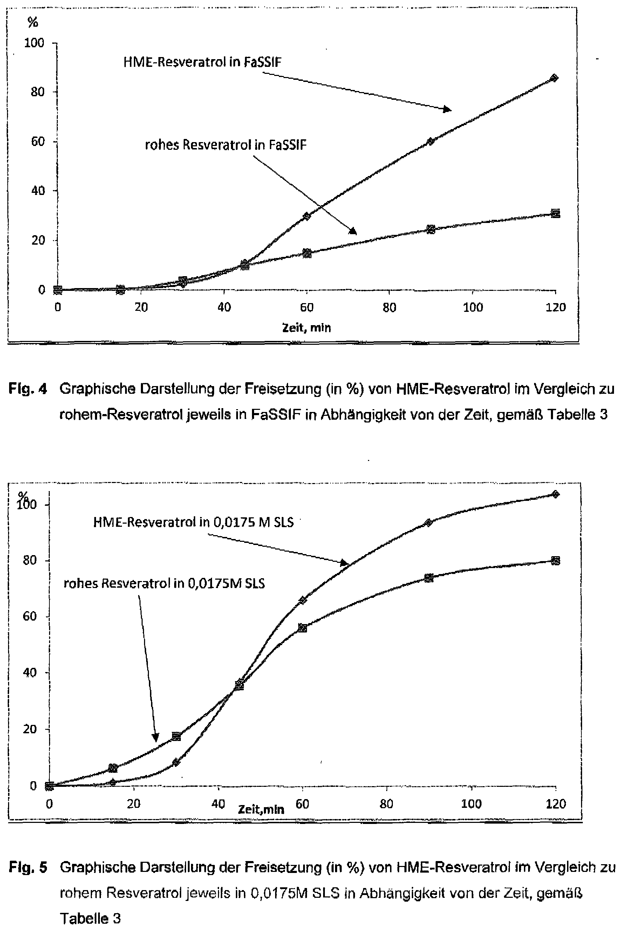

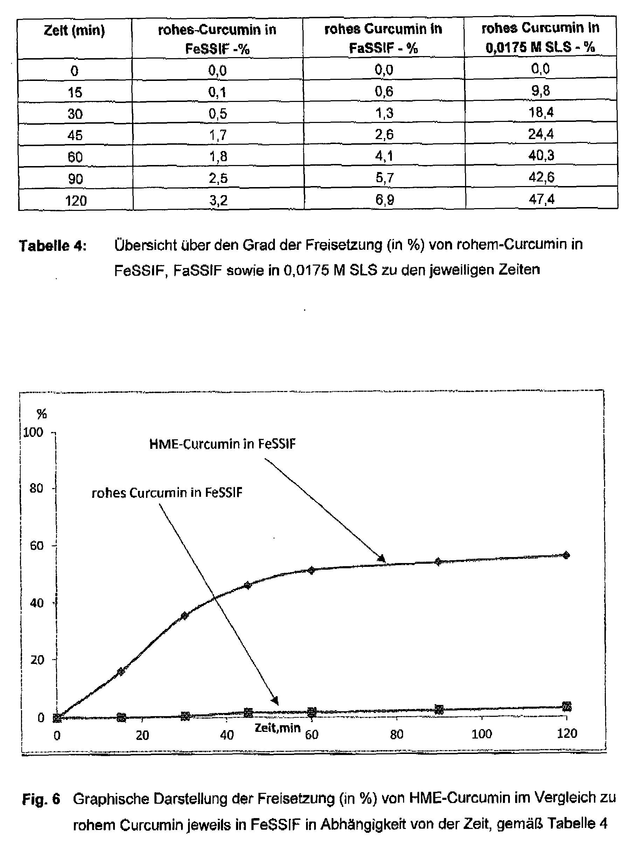

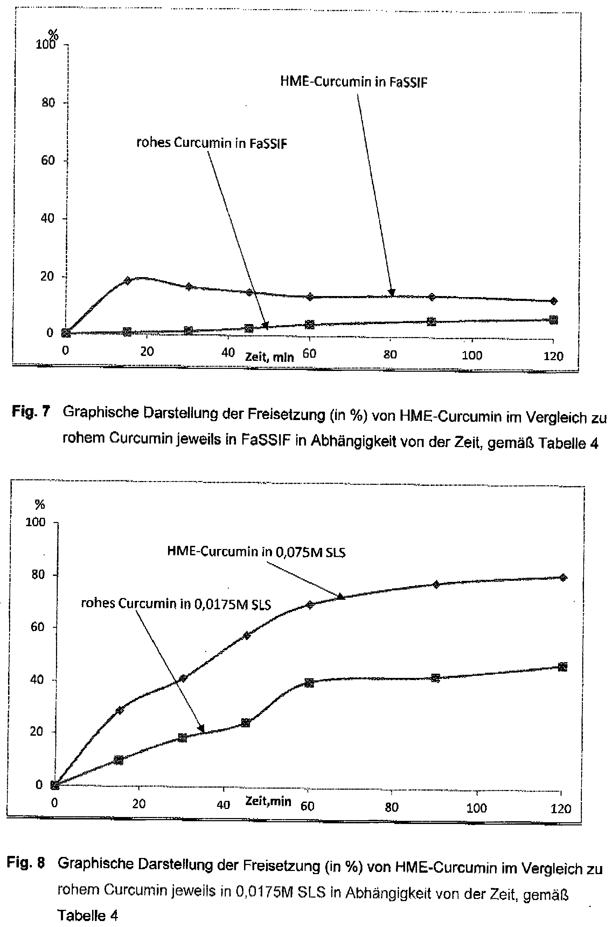

Die Ergebnisse sind in den Tabellen 1 bis 4 und den Figuren 1 bis 8 dargestellt.

Die Tabellen 1 bis 4 und die Figuren 1 bis 8 zeigen, dass die Löslichkeit von Resveratrol und Curcumin wesentlich von den Bedingungen im menschlichen Körper abhängt, unter denen sich die Auflösung vollziehen soll.

Besonders augenscheinlich ist, dass der Grad der Löslichkeit des Resveratrol und Curcumins aus der mittels HME extrudierten erfinderischen Zusammensetzung im Darmbereich jeweils signifikant höher ist als der von rohem Resveratrol und rohem Curcumin in ihren Dispersionen. Die so belegte signifikant höhere Löslichkeit im Darmbereich ist Voraussetzung für die signifikant höhere Bioverfügbarkeit der beiden Wirkstoffe im menschlichen Organismus und belegt den besonderen Vorzug der Erfindung überzeugend.

Bisher verhinderte die schnelle Metabolisierung der Wirkstoffe bei oraler Einnahme nicht extrudierter Festkörperdispersionen sowie anderer nicht extrudierter Darreichungsformen, dass Resveratrol und Curcumin überhaupt den Darm in einer therapeutisch wirksamen Menge erreichen. Durch das erfindungsgemäße Extrudat wird zugleich auch das Problem der Metabolisierung gelöst und ein weiterer besonderer Vorzug der Erfindung überzeugend belegt.

Die verhinderte Metabolisierung, die erreichte hohe Löslichkeit und die dadurch bedingte signifikant höhere Bioverfügbarkeit im Darmbereich des Menschen sind von weitreichender Bedeutung, zumal sie altersunabhängig sind. Die durch die Wissenschaft bekannten therapeutischen Wirkungen von Resveratrol und Curcumin werden durch deren synergistisches Wirken gemäß Erfindung auch auf ökonomisch vorteilhafte Weise wesentlich unterstützt.

Im Folgenden soll das Potenzial der erfindungsgemäßen Zusammensetzung für die Ausbildung pharmazeutisch wirksamer Zusammensetzungen und insbesondere für deren orale Verabreichung verdeutlicht werden. Die folgenden Ausführungen sind so dargestellt, dass sie denjenigen, die über gewöhnliche Fertigkeiten auf dem betreffenden Gebiet verfügen, das der Erfindung innewohnende Potenzial, vor allem deren Verwendungsbreite näher bringen, ohne die Erfindung und deren Umfang einzuschränken.

Die Einführung neuartiger Polymere für HME wird dazu beitragen, die derzeitigen Einschränkungen durch Unverträglichkeiten zwischen Medikamenten und Polymermatrix zu beseitigen.

In klinischen Standardversuchen wurde die Eignung und Wirksamkeit pharmazeutischer Zusammensetzungen untersucht, die unter Verwendung der erfindungsgemäßen Zusammensetzung hergestellt wurden. Zur Anwendung kamen Verabreichungen in Form von Tabletten, Kapseln, Flüssigkeiten, Sirupen oder Pulvern (in Beuteln) oder dispergierbaren Tabletten zur Suspension, die dem Wasser zugesetzt wurden, die therapeutisch wirksame Mengen der Erfindung aufweisen und diese im Blut abgeben. Die Dosen lagen im Bereich von 2,5 mg bis 250 mg der therapeutisch wirksamen Verbindung pro Tag für Menschen mit einem Körpergewicht von 75 kg, beispielsweise bei einem Erwachsenen sowie mit Hilfe von Standard-Tiermodellen.

In einzelnen Fällen enthält die pharmazeutisch wirksame Zusammensetzung Dosen mit Partikeln, die nicht pH-resistent sind ober mit Partikeln, die pH-resistent sind und/oder ein verzögertes Zeit- und Enzymfreisetzungsprofil aufweisen.

Resveratrol besitzt eine breite Palette biologischer Eigenschaften, darunter antioxidative, kardioprotektive, neuroprotektive, entzündungshemmende, krebsbekämpfende und blutzuckersenkende Eigenschaften sowie lebensverlängernde Effekte. Auf dem Markt angeboten und eingesetzt wird traditionell Resveratrol bislang bei Magenschmerzen, Hepatitis, Arthritis, Harnwegsinfektionen, Pilzerkrankungen oder zur Behandlung von Hautentzündungen.

Das biologische Potenzial von Resveratrol liegt jedoch hauptsächlich im Bereich von kardioprotektiven Systemen, in der Behandlung von arteriosklerotischen Herz-Kreislauf-Erkrankungen, sowie von entzündlichen Nerven- und von onkologischen Erkrankungen, des Weiteren im Bereich von epigenetischen Modifikationen.

Resveratrol ist in der Lage, alle Karzinogenese-Stadien zu hemmen (z.B. Beginn, Förderung und Fortschreiten der Krebserkrankung) und das darüber hinaus nicht nur als chemopräventives Mittel wirkt, sondern auch chemotherapeutische Eigenschaften aufweist, die mit seinen entzündungshemmenden, antioxidativen, pro-apoptotischen und antiproliferativen Wirkungen verbunden sind.

Im Besonderen ist die vorliegende Erfindung auf die Verwendung für pharmazeutisch wirksame Zusammensetzungen zur therapeutischen Behandlung arteriosklerotische Herz-Kreislauf-Erkrankungen sowie von entzündlichen Nerven- und onkologischen Erkrankungen ausgerichtet.

Abgesehen von diesen Anwendungen ist Resveratrol wie dessen Derivate eine der vielversprechendsten Verbindungen auf dem Gebiet der entzündungshemmenden Arzneimittelforschung, Es hat sich auch gezeigt, dass es in der Lage ist, kalorische Restriktionseffekte zu initiieren und dass es über verschiedene Mechanismen verfügt, um den Beginn und das Fortschreiten vieler Krankheiten zu beeinflussen,

Zwar gibt es eine Fülle von vitro- und vivo-Beweisen, dass Resveratrol ein vielversprechendes Therapeutikum sein könnte, doch müssen weitere klinische Studien das durch die gefundene höhere Löslichkeit und Bioverfügbarkeit gegebene Potenzial von Resveratrol noch umfassender und genauer bestätigen, insbesondere auch im synergistischen Wirken von Resveratrol zusammen mit Curcumin.

Curcumin hat weltweit Aufmerksamkeit für seine vielfältigen gesundheitlichen Vorteile erhalten, die offenbar in erster Linie durch seine antioxidativen und entzündungshemmenden Mechanismen wirken. Diese Vorteile werden am besten erreicht, wenn Curcumin mit Wirkstoffen wie Piperin kombiniert wird, die seine Bioverfügbarkeit noch weiter erhöhen. Forschungsergebnisse deuten darauf hin, dass Curcumin bei der Behandlung von oxidativen und entzündlichen Zuständen, metabolischem Syndrom, Arthritis, Angstzuständen und Hyperlipidämie helfen kann. Es kann auch bei der Behandlung von durch körperliche Anstrengung verursachten Entzündungen und Muskelkater helfen und so die Erholung und die anschließende Leistung bei aktiven Menschen verbessern. Darüber hinaus kann eine relativ niedrige Dosis gesundheitliche Vorteile für Menschen bieten, bei denen keine gesundheitlichen Probleme diagnostiziert wurden.

Erfindungsgemäße Zusammensetzungen von Resveratrol und Curcumin als wesentlicher Bestandteil pharmazeutischer Zusammensetzungen und in entsprechenden Darreichungsformen sind hochwirksam in der Prävention und Behandlung verschiedener Krebsarten, nämlich unter entzündungshemmenden, antikanzerogenen, kardioprotektiven, gefäßschützenden, gefäßerweiternden, phytoöstrogenen, neuroprotektiven und entzündlichen Aspekten.

Neben ihrer hohen Wirksamkeit, die erreicht wird durch die verbesserte Löslichkeit in wässrigen Medien und die damit signifikant erhöhte Bioverfügbarkeit, verursachen die erfindungsgemäßen Zusammensetzungen auch deutlich weniger Nebenwirkungen als die derzeit verfügbaren pharmazeutischen und pflanzlichen Wirkstoffe und Wirkstoffkombinationen. Sie können daher als Ersatz für diese existierenden synthetischen und pflanzliche Wirkstoffe und Wirkstoffkombinationen eingesetzt werden.

Sie helfen bei der Behandlung von oxidativen und entzündlichen Zuständen, metabolischem Syndrom, Arthritis, Angstzuständen und Hyperlipidämie.

## Patentansprüche

1. Verfahren zur Herstellung einer Zusammensetzung, enthaltend Resveratrol und Curcumin als natürliche lipophile Verbindungen, wobei die Zusammensetzung außerdem enthält:
- mindestens einen nichtionischen Emulgator und/oder mindestens einen anionischen Emulgator, wobei mindestens ein Emulgator ausgewählt ist aus nichtionischen oder anionischen Tensiden mit einem Hydrophilic-Balance-Wert (HLB-Wert) von etwa 10 bis etwa 16 und ein zweiter Emulgator ausgewählt ist aus nichtionischen oder anionischen hydrophilen oder hydrophoben Tensiden mit einem HLB-Wert von etwa 4 bis etwa 12, und
- mindestens ein festes matrixbildendes Mittel, ausgewählt aus der Gruppe bestehend aus Polyvinylcaprolactam-Polyvinylacetat-Polyethylenglykol-Pfropfcopolymer (Soluplus^{®}), Polyvinylalkohol (PVA), vernetztem Copolymer aus Acrylsäure und einem hydrophoben C10-30-Alkylacrylat-Comonomer, Gelatine, Hydroxypropylmethylcellulose (Methocel), Methylcellulose, Hydroxypropylcellulose (Klucel), Hydroxyethylcellulose (Natrosol), Natriumcarboxymethylcellulose, Acrylat-Copolymere, Ammonio-Methacrylat-Copolymer Typ A oder B, Dimethylaminoethylmethacrylat-Butylmethacrylat-Methylmethacrylat-Copolymer, Methacrylsäure-Ethylacrylat-Copolymer, Polyvinylalkohol-Pfropfcopolymer, Polyvinylacetat-Co-Crotonsäure, Polymethylmethacrylat und gepfropftes Polyethylenoxid, Lignine, Polyvinylpyrrolidon-Co-Vinylacetat, Polyvinylpyrrolidon, Celluloseacetatphthalat, Hydroxypropylmethylcellulosephthalat, Carboxymethylethylcellulose, Hydroxypropylmethylcelluloseacetatsuccinat, Polyvinylacetat-Phtalat, Maltodextrin, Dextran, Polymethacrylsäure-Co-Ethylacrylat, Polymethacrylsäure-Co-Methylmethacrylat, Polylactidsäure (PLA), Poly-L-Lactid (PLLA), Poly-D-Lactid (PDLA), Polylactid-Co-Glykolsäure (PLGA), Polyethylenoxid-Polypropylenoxid-Blockcopolymer (Poloxamer), Polyethylenglykol (PEG1000, PEG1500, PEG 2000, PEG4000, PEG6000, PEG8000) und aus Mischungen davon,
- wobei Resveratrol und Curcumin zusammen mit mindestens einem Emulgator und mindestens einem matrixbildenden Mittel in einem festen Lösungsmittelsystem löslich ausgebildet sind, wobei mindestens 80 % des Resveratrols und des Curcumins, gemessen mit Differential-Scanning-Kalorimetrie, in der festen Zusammensetzung vollständig gelöst sind,
wobei das Verfahren **dadurch gekennzeichnet ist, dass**
- das Herstellen der Zusammensetzung mittels Heißschmelzextrusion erfolgt, umfassend die Schritte des Zuführens der Bestandteile in einen Heißschmelzextruder, des Mischens und Erhitzens der Bestandteile bis zum Schmelzen und das anschließende Extrudieren, Abkühlen und Formen, wobei alle diese Schritte zusammen einen einzigen kontinuierlichen Prozess bilden.

2. Verfahren nach Anspruch 1, **dadurch gekennzeichnet, dass** das Resveratrol der Formel 3,5,4'-trihydroxy-trans-stilben ausgewählt ist aus der Gruppe der Familien Dipterocarpaceae, Paeoniaceae, Vitaceae, Leguminosae, Gnetaceae, Cyperaceae, Polygonaceae Gramineae und Poaceae und das Curcumin die Formel (1E,6E)-1,7-Bis (4-hydroxy-3-methoxyphenyl) -1,6-heptadien-3,5-dione aufweist.

3. Verfahren nach Anspruch 1, **dadurch gekennzeichnet, dass** mindestens ein nichtionischer Emulgator, ausgewählt ist aus der Gruppe, die aus Polysorbaten, Polysorbat 80, polyoxylhydriertem Rizinusöl, Saccharoseester, Saccharosedistearat, Tocopherylpolyethylenglykol 1000-Succinat, Sorbitanfettsäureester, Sorbitanmonooleat, Polyglycerylfettsäureester, Polyoxylglyceriden oder Salzen, Derivaten oder Kombinationen davon besteht.

4. Verfahren nach Anspruch 1, **dadurch gekennzeichnet, dass** ein zweites festes matrixbildendes Mittel, ausgewählt ist aus der Gruppe bestehend aus Bienenwachs, Carnaubawachs, Cetylpalmitat, Glycerylbehenat, Behensäure, Behenylalkohol, Glycerylmonostearat, Glycerylpalmitostearat, Glycerylstéarat, hydriertes Rizinusöl, mikrokristallines Wachs, Paraffinwachs, Stearinsäure, Stearinsäurealkohol, Alkylsilikon, Silikonwachs, wachsartiges Polymethylsiloxan, PEG-Wachs und Carbowax.

5. Verfahren nach mindestens einem der Ansprüche 1 bis 4, **dadurch gekennzeichnet, dass** das Verhältnis des Resveratrol und des Curcumins zu mindestens einem Emulgator von etwa 5:1 bis etwa 1:20 beträgt.

6. Verfahren nach mindestens einem der Ansprüche 1 bis 5, **dadurch gekennzeichnet, dass** das Verhältnis von a) dem Resveratrol und dem Curcumin zu b) der Mischung aus dem mindestens einen Emulgator und dem mindestens einem festen matrixbildenden Mittel etwa 2:1 bis etwa 1:20 beträgt.

7. Verfahren nach Anspruch 1, **dadurch gekennzeichnet, dass** die Zusammensetzung enthält
- Resveratrol mit einer Reinheit von 99,84 % zu 16,67 %
- Curcumin mit einer Reinheit von 95,5 % zu 17,50 %
- Polyethylenglykol 2000 zu 3,33 %
- Poloxamer 407 zu 30,00 %
- β-Cyclodextrin zu 10,00 %
- Vitamin E TPGS zu 4,17 %
- Compritol 888 ATO zu 1,67 %
- Aerosil 200 zu 2,50 % und
- Polyvinylalkohol PVA zu 14,17 %.

8. Verfahren nach einem der Ansprüche 1 bis 7, **dadurch gekennzeichnet, dass** die Heißschmelzextrusion bei 80 U/min und einem Vorschub von 1 kg/h durchgeführt und die Temperatur mit 105° ± 1°C gehalten wird.

## Claims

1. Method for preparing a composition comprising resveratrol and curcumin as natural lipophilic compounds, the composition also comprising:
- at least one nonionic emulsifier and/or at least one anionic emulsifier, wherein at least one emulsifier is selected from nonionic or anionic surfactants having a hydrophilic-lipophilic balance value (HLB value) of from about 10 to about 16 and a second emulsifier is selected from nonionic or anionic hydrophilic or hydrophobic surfactants having an HLB value of from about 4 to about 12, and
- at least one solid matrix-forming agent selected from the group consisting of polyvinyl caprolactam-polyvinyl acetate-polyethylene glycol graft copolymer (Soluplus^{®}), polyvinyl alcohol (PVA), crosslinked copolymer of acrylic acid and a hydrophobic C10-C30 alkyl acrylate comonomer, gelatin, hydroxypropyl methylcellulose (Methocel), methylcellulose, hydroxypropylcellulose (Klucel), hydroxyethylcellulose (Natrosol), sodium carboxymethylcellulose, acrylate copolymers, type A or B ammonium methacrylate copolymer, dimethylaminoethyl methacrylate-butyl methacrylate-methyl methacrylate copolymer, methacrylic acid-ethyl acrylate copolymer, polyvinyl alcohol graft copolymer, poly(vinyl acetate-co-crotonic acid), poly(methyl methacrylate) and grafted polyethylene oxide, lignins, poly(vinylpyrrolidone-co-vinyl acetate), polyvinylpyrrolidone, cellulose acetate phthalate, hydroxypropyl methylcellulose phthalate, carboxymethyl ethylcellulose, hydroxypropyl methylcellulose acetate succinate, polyvinyl acetate phthalate, maltodextrin, dextran, poly(methacrylic acid-co-ethyl acrylate), poly(methacrylic acid-co-methyl methacrylate), polylactic acid (PLA), poly-L-lactic acid (PLLA), poly-D-lactic acid (PDLA), poly(lactic-co-glycolic acid) (PLGA), polyethylene oxide-polypropylene oxide block copolymer (Poloxamer), polyethylene glycol (PEG1000, PEG1500, PEG 2000, PEG4000, PEG6000, PEG8000) and mixtures thereof,
- wherein resveratrol and curcumin are made soluble in a solid solvent system together with at least one emulsifier and at least one matrix-forming agent, wherein at least 80% of the resveratrol and curcumin, measured by differential scanning calorimetry, is completely dissolved in the solid composition,
wherein the method is **characterized in that**
- the composition is prepared by hot-melt extrusion, comprising the steps of supplying the constituents to a hot-melt extruder, mixing, and heating the constituents until they melt, and the subsequent extrusion, cooling and forming, all these steps together forming a single continuous process.

2. Method according to Claim 1, **characterized in that** the resveratrol of formula 3,5,4'-trihydroxy-trans-stilbene is selected from the group of the families Dipterocarpaceae, Paeoniaceae, Vitaceae, Leguminosae, Gnetaceae, Cyperaceae, Polygonaceae Gramineae, and Poaceae and that the curcumin has the formula (1E,6E)-1,7-bis(4-hydroxy-3-methoxyphenyl)-1,6-heptadiene-3,5-dione.

3. Method according to Claim 1, **characterized in that** at least one nonionic emulsifier is selected from the group consisting of polysorbates, polysorbate 80, polyoxyl hydrogenated castor oil, sucrose esters, sucrose distearate, tocopheryl polyethylene glycol 1000 succinate, sorbitan fatty acid esters, sorbitan monooleate, polyglycerol fatty acid esters, polyoxyl glycerides, or salts, derivatives or combinations thereof.

4. Method according to Claim 1, **characterized in that** a second solid matrix-forming agent is selected from the group consisting of beeswax, carnauba wax, cetyl palmitate, glyceryl behenate, behenic acid, behenyl alcohol, glyceryl monostearate, glyceryl palmitostearate, glyceryl stearate, hydrogenated castor oil, microcrystalline wax, paraffin wax, stearic acid, stearic alcohol, alkyl silicone, silicone wax, waxy polymethylsiloxane, PEG wax and Carbowax.

5. Method according to at least one of Claims 1 to 4, **characterized in that** the ratio of the resveratrol and the curcumin to the at least one emulsifier is from about 5:1 to about 1:20.

6. Method according to at least one of Claims 1 to 5, **characterized in that** the ratio of a) the resveratrol and the curcumin to b) the mixture of the at least one emulsifier and the at least one solid matrix-forming agent is from about 2:1 to about 1:20.

7. Method according to Claim 1, **characterized in that** the composition comprises
- resveratrol with a purity of 99.84% to 16.67%
- curcumin with a purity of 95.5% to 17.50%
- polyethylene glycol 2000 in a content of 3.33%
- Poloxamer 407 in a content of 30.00%
- β-cyclodextrin in a content of 10.00%
- vitamin E TPGS in a content of 4.17%
- Compritol 888 ATO in a content of 1.67%
- Aerosil 200 in a content of 2.50% and
- polyvinyl alcohol PVA in a content of 14.17%.

8. Method according to any of Claims 1 to 7, **characterized in that** the hot-melt extrusion is carried out at 80 rpm and a feed rate of 1 kg/h and that the temperature is maintained at 105°C ± 1°C.

## Revendications

1. Procédé de préparation d'une composition contenant du resvératrol et de la curcumine en tant que composés lipophiles naturels, la composition contenant en outre :
- au moins un émulsifiant non ionique et/ou au moins un émulsifiant anionique, au moins un émulsifiant étant choisi parmi les tensioactifs non ioniques ou anioniques ayant une valeur d'équilibre hydrophile (valeur HLB) d'environ 10 à environ 16 et un deuxième émulsifiant étant choisi parmi les tensioactifs hydrophiles ou hydrophobes non ioniques ou anioniques ayant une valeur HLB d'environ 4 à environ 12, et
- au moins un agent solide générateur de matrice, choisi dans le groupe constitué par le copolymère greffé polyvinylcaprolactame-poly(acétate de vinyle)-polyéthylèneglycol (Soluplus^{®}), le poly(alcool vinylique) (PVA), un copolymère réticulé à base d'acide acrylique et d'un comonomère acrylate d'alkyle en C10-30 hydrophobe, la gélatine, l'hydroxypropylméthylcellulose (Methocel), la méthylcellulose, l'hydroxypropylcellulose (Klucel), l'hydroxyéthylcellulose (Natrosol), la carboxyméthylcellulose sodique, les copolymères d'acrylate, le copolymère ammonio-méthacrylate de type A ou B, le copolymère méthacrylate de diméthylaminoéthyle-méthacrylate de butyle-méthacrylate de méthyle, le copolymère acide méthacrylique-acrylate d'éthyle, un copolymère greffé de poly(alcool vinylique), le copolymère acétate de vinyle-acide crotonique, le poly(méthacrylate de méthyle) et poly(oxyde d'éthylène) greffé, les lignines, le copolymère vinylpyrrolidone-acétate de vinyle, la polyvinylpyrrolidone, l'acétate-phtalate de cellulose, le phtalate d'hydroxypropylméthylcellulose,
la carboxyméthyléthylcellulose, l'acétate-succinate d'hydroxypropylméthylcellulose, le poly(acétate-phtalate de vinyle), la maltodextrine, le dextrane, le copolymère acide méthacrylique-acrylate d'éthyle, le copolymère acide méthacrylique-méthacrylate de méthyle, le poly(acide lactique) (PLA), le poly-L-lactide (PLLA), le poly-D-lactide (PDLA), le polylactide-co-acide glycolique (PLGA), le copolymère à blocs polyoxyéthylène-polyoxypropylène (Poloxamer), le polyéthylèneglycol (PEG1000, PEG1500, PEG 2000, PEG4000, PEG6000, PEG8000) et des mélanges de ceux-ci,
- le resvératrol et la curcumine, conjointement avec au moins un émulsifiant et au moins un agent générateur de matrice, étant conçus pour être solubles dans un système de solvant solide, au moins 80 % du resvératrol et de la curcumine, mesurés par calorimétrie différentielle à balayage, étant totalement dissous dans la composition solide,
le procédé étant **caractérisé en ce que**
- la préparation de la composition s'effectue par extrusion à chaud de masse fondue, comprenant les étapes de l'introduction des constituants dans une extrudeuse de masse fondue à chaud, du mélange et du chauffage des constituants jusqu'à la fusion et l'extrusion, le refroidissement et le formage subséquents, toutes ces étapes constituant ensemble un seul processus continu.

2. Procédé selon la revendication 1, **caractérisé en ce que** le resvératrol de formule 3,5,4'-trihydroxy-trans-stilbène est choisi dans le groupe des familles Dipterocarpaceae, Paeoniaceae, Vitaceae, Leguminosae, Gnetaceae, Cyperaceae, Polygonaceae, Gramineae et Poaceae et la curcumine présente la formule (1E,6E)-1,7-bis (4-hydroxy-3-méthoxyphényl)-1,6-heptadiène-3,5-dione.

3. Procédé selon la revendication 1, **caractérisé en ce qu'**au moins un émulsifiant non ionique est choisi dans le groupe constitué par les polysorbates, le polysorbate 80, l'huile de ricin polyoxyhydrogénée, les esters de saccharose, le distéarate de saccharose, le succinate de tocophérylpolyéthylèneglycol 1000, les esters d'acides gras et de sorbitane, le monooléate de sorbitane, les esters d'acides gras et de polyglycéryle, les polyoxyglycérides ou les sels, dérivés ou associations de ceux-ci.

4. Procédé selon la revendication 1, **caractérisé en ce qu'**un deuxième agent solide générateur de matrice est choisi dans le groupe constitué par la cire d'abeille, la cire de carnauba, le palmitate de cétyle, le béhénate de glycéryle, l'acide béhénique, l'alcool béhénylique, le monostéarate de glycéryle, le palmitostéarate de glycéryle, le stéarate de glycéryle, l'huile de ricin hydrogénée, la cire microcristalline, la cire de paraffine, l'acide stéarique, l'alcool stéarylique, une alkylsilicone, la cire de silicone, le polyméthylsiloxane cireux, la cire de PEG et le Carbowax.

5. Procédé selon au moins l'une quelconque des revendications 1 à 4, **caractérisé en ce que** le rapport du resvératrol et de la curcumine à au moins un émulsifiant vaut d'environ 5:1 à environ 1:20.

6. Procédé selon au moins l'une quelconque des revendications 1 à 5, **caractérisé en ce que** le rapport de a) le resvératrol et la curcumine à b) le mélange dudit au moins un émulsifiant et dudit au moins un agent solide générateur de matrice vaut d'environ 2:1 à environ 1:20.

7. Procédé selon la revendication 1, **caractérisé en ce que** la composition contient
- du resvératrol ayant une pureté de 99,84 %, à 16,67 %
- de la curcumine ayant une pureté de 95,5 %, à 17,50 %
- du polyéthylèneglycol 2000, à 3,33 %
- du Poloxamer 407, à 30,00 %
- de la β-cyclodextrine, à 10,00 %
- de la vitamine E TPGS, à 4,17 %
- du Compritol 888 ATO, à 1,67 %
- de l'Aerosil 200, à 2,50 % et
- du poly(alcool vinylique) PVA, à 14,17 %.

8. Procédé selon l'une quelconque des revendications 1 à 7, **caractérisé en ce que** l'extrusion de masse fondue à chaud est effectuée à 80 tours/min et à une avance de 1 kg/h, et la température est maintenue à 105 °C ± 1 °C.
